# EUROPEAN PATENT APPLICATION

(11) **EP 1 400 212 A1**
(43) Date of publication of application: **24.03.2004**
(21) Application number: 02738808.1
(22) Date of filing: 26.06.2002
(51) Int. Cl.: A61B 17/56

(54) **APPARATUS AND METHOD FOR TREATING JOINT DISEASE**

(30) Priority: 26.06.2001 JP 2001192629
(71) Applicant: TEIJIN LIMITED, Osaka-shi Osaka 541-0054 (JP)
(72) Inventor: ITO, Masaya, Teijin Limited, Tokyo Research Center, Hino-shi, Tokyo 191-0065 (JP); AZUMA, Yoshiaki, Teijin Limited, Tokyo Res. Center, Hino-shi, Tokyo 191-0065 (JP); OHTA, Tomohiro, c/o Teijin Limited, Tokyo 100-0011 (JP); TAKAICHI, Satoshi, Fujisawa-shi, Kanagawa 251-0861 (JP); SUZUKI, Seiichiro, Teijin Ltd, Tech Serv Cntr Home, Anpachi-gun, Gifu 50 (JP)
(74) Representative: HOFFMANN EITLE
(86) International application number: PCT/JP2002/006438
(87) International publication number: WO 2003/000147

(57) **Abstract**

The present invention provides an apparatus and a method for treating joint diseases. In order to lower a matrix metalloprotease (MMP) activity which has been enhanced in accompany with a joint disease such as osteoarthritis or rheumatoid arthritis, and to improve the joint disease, the apparatus lowers the MMP activity at the joint site by applying ultrasonic waves on it. The apparatus is a means provided with at least an ultrasonic transducer, an ultrasonic generator and a means for mounting the ultrasonic transducer on the joint site, and applying ultrasonic waves having a frequency of 1.3 to 2 MHz, a repeating frequency of 100 to 1,000 Hz, a burst width of 10 to 2,000 µs and an intensity of 100 mW/cm² or less (SATA: Spatial Average-Temporal Average).

## Description

### Technical Field

The present invention relates to an apparatus and a method for treating joint diseases. More specifically, the invention relates to an apparatus which applies ultrasonic waves to a joint disease site to lower matrix metalloprotease (MMP) activity in synovia in a joint disease, suppress tissue degradation and at the same time improve joint tissues, and to a method for the same.

### Background Art

Major joint diseases include rheumatoid arthritis, osteoarthritis and the like. These joint diseases have large differences from each other in etiologies and morbid conditions, but they are same to each other in a point that they accompany inflammations, pains, motion failure etc. due to the damage of articular cartage and finally cause joint function disorder. It is the present state of joint diseases that the number of patients is increasing, but there is no efficient treating agent capable of surely completely curing them although a treating agent corresponding to the morbid condition, for example, an analgesic antiphlogistic such as aspirin or indomethacin, a steroid drug, an immunomodulator or the like, is commonly administered.

A matrix metalloprotease (MMP) is a protease having a metal ion on the active center, and it is suggested that MMPs play a part in the morbid condition of joint diseases such as osteoarthritis [Maiotti et al., Arthroscopy 16, 522-526 (2000); and Tanaka, et at., Semin Arthritis Rheum 27, 392-399 (1998)].

It is known that there are many types of MMP having different enzymes. Among them, MMP-1 is called collagenase-1, and has an activity to degrade collagen, and it is known for a long time that MMP-1 is involved in osteoarthritis [Ehrlich et al., J. Clin. Inves., 59, 226-233 (1977); and Pelletier et al., Arthritis Rheum., 26, 63-68 (1983)].

Further, regarding the relation between MMP-3 (stromelysin-1), which has an activity to decompose an aggrecan, a cartilage matrix, and osteoarthritis, Okada et al., reported that MMP-3 was deeply involved in chondrodegeneration in osteoarthritis by showing a good correlation between an incidence rate of MMP-3 positive chondrocytes and a degree of chondrodegeneration [Okada et al., Lab. Invest., 66, 680-690 (1992)].

Furthermore, MMP-13 called also collagenase-3 has an activity to decompose collagen more quickly than MMP-1, and it was suggested that MMP-13 was involved in cartilage degradation in osteoarthritis [Mitchell et al., J. Clin. Invest., 97, 761-768 (1996)]. It was also reported that an MMP activity was increased in the synovia of a patient of rheumatoid arthritis.

Agents having an MMP activity-inhibiting action are expected to be useful as a treating agent for rheumatoid arthritis, osteoarthritis or the like [Natchus et al., J. Med. Chem., 43, 4948-4963 (2000); and Bender et al., United State Patent No. 6,174,915]. An administration of an antagonist of an interleukin-1 (IL-1) receptor is known as a method for improving a tissue score in a joint disease (Pelletier et al., United State Patent No. 5,972,880).

Although pharmacotherapy most frequently using an MMP inhibitor against a joint disease is being studied, a medicine having sufficient effect as a treating agent has not been developed yet.

On the other hand, a treating method for joint diseases by physical stimulation is developed as physiotherapy, and a treating method for osteoarthritis characterized in that pain is alleviated by applying electric dermatological stimulation is known (Hoffman, United State Patent No. 5,273,033). However, the method needs such a long electric stimulation as 8 hours a day, and it has a room for improvement, taking consideration of a binding hour on the patient during treatment, and it is desired to development a method and an apparatus for treating in a short time. Further, regarding actual methods for suppressing a joint tissue degeneration and decreasing MMP activity in synovia., nothing is known, and it is wanted to develop a method and an apparatus for treating a joint disease by improving the tissue score through effectively decreasing MMP activity.

### Disclosure of the Invention

The above purposes are achieved by the present invention. The inventors of the present invention pursued zealous studies on methods for suppressing the increase of MMP activity accompanied by a joint disease and for improving the joint disease, and they found the following method.

That is, the present invention provides a joint disease-treating apparatus. The apparatus lowers MMP activities at a joint disease site by applying ultrasonic waves to the joint site. It is a means comprising at least an ultrasonic transducer, an ultrasonic generator and a means for mounting the ultrasonic transducer on the joint site, and applying ultrasonic waves having a frequency of 1.3 to 2 MHz, a repeating frequency of 100 to 1,000 Hz, a burst width of 10 to 2,000 µs and an output of 100 mW/cm² or less (SATA: Spatial Average-Temporal Average).

Further, the present invention provides a method for decreasing MMP activities by applying ultrasonic waves on a site where MMP activities are enhanced. Especially, it is characterized that the ultrasonic waves have a frequency of 1.3 to 2 MHz, a repeating frequency of 100 to 1,000 Hz, a burst width of 10 to 2,000 µs and an output of 100 mW/cm² or less (SATA: Spatial Average-Temporal Average), and the MMP is MMP-1, MMP-3 and/or MMP-13.

### Brief Description of drawings

Figure 1 is a figure showing the effect of low intensity ultrasonic waves on femoral condyle of the knee joint based on an evaluation with a modified Mankin score. The data are shown by an averaged value ± a standard deviation. The p-value in the figure is the result of the unpaired one-tailed Student's t-test on the difference between two groups.
Figure 2 is a figure showing the effect of low output ultrasonic waves applied on an MMP-1 activity in the synovia.. The date are expressed by an average ± a standard deviation. The p-value in the figure is the result of the unpaired one-tailed Student's t-test on the difference between two groups.
Figure 3 is a figure showing the effect of low output ultrasonic waves applied on an MMP-3 activity in the synovia.. The datum is expressed by an average ± a standard deviation. The p-value in the figure is the result of the unpaired one-tailed Student's t-test on the difference between two groups.
Figure 4 is a figure showing the effect of low intensity ultrasonic waves applied on an MMP-13 activity in the synovia. The datum is expressed by an average ± a standard deviation. The p-value in the figure is the result of the unpaired one-tailed Student's t-test on the difference between two groups.
Figure 5 is a sketch drawing of a joint disease treating apparatus of the present invention.
Figure 6 is a rough flow sheet of a joint disease treating apparatus of the present invention.

### Best Mode for Carrying Out the Invention

Hereafter, the present invention will be explained in detail.

A joint disease treating apparatus of the present invention is an apparatus capable of decreasing the MMP activity at a joint site by the application of ultrasonic waves, and the apparatus comprises at least an ultrasonic transducer, an ultrasonic generator and a means for mounting the ultrasonic transducer on the joint site.

The ultrasonic waves to be applied are such low output ultrasonic waves that heat is hardly generated. Concretely, they are low intensity ultrasonic waves having a frequency of 1.3 to 2 MHz, a repeating frequency of 100 to 1,000 Hz, a burst width of 10 to 2,000 µs and an output of 100 mW/cm² or less (SATA). As for an application method, the ultrasonic waves are preferably applied continuously in such a manner that low output ultrasonic pulses are transmitted to a disease site, for example, for 20 minutes a day. It is preferable that, for example, an ultrasonic wave output unit (SAFHS 2000®) manufactured by Exogen Co., in the United States is used, and the ultrasonic waves have characteristics of a burst width of 200 µs, a frequency of 1.5 MHz, a repeating frequency of 1 kHz and an output of 30 mW/cm².

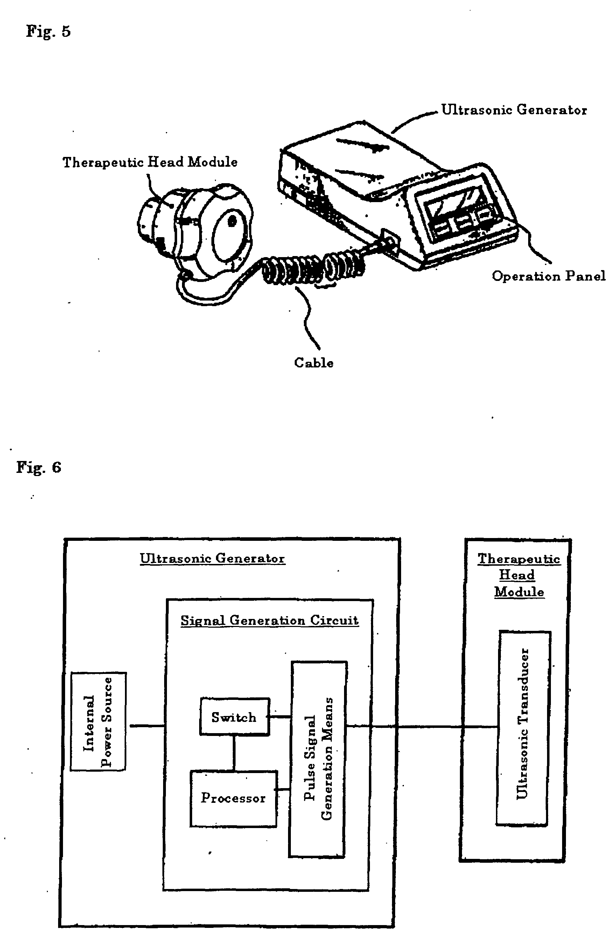

A construction of the ultrasonic generator and the ultrasonic transducer are shown by Figure 5 and Figure 6, and the joint disease treating apparatus is a noninvasive treating apparatus in which signals from the ultrasonic generator are transmitted to a treating head module having a built-in transducer via a cable, and the ultrasonic pulses having the above-mentioned characteristics are applied transdermally from the outside part of the body adjacent to the disease site.

Methods for mounting the treating head module on a joint site of a knee or an elbow differ according to a disease site or morbid conditions. However, in the case of a trouble at a loading part of a femoral condyle, the head module is fixed by the thigh and/or the lower limb, and at the same time the mounting means has a joint angle fixing part for fixing a joint in a state where the joint is bent squarely and a mounting part for the ultrasonic treating head module. The module mounting part is placed so that ultrasonic waves are applied in the direction of the joint cavity of the limb and in the direction of the loading part of the femoral condyle. For example, a joint mounting fixture described in WO 00/47125 is preferably used.

The irradiation of ultrasonic waves can be performed without having attenuation by bringing the treating head module into contact with the skin via an ultrasonic transmitting gel.

The joint disease on which the joint disease treating apparatus of the present invention is applied is a disease in which an MMP activity has been enhanced, and such joint diseases include osteoarthritis, osteochondritis dissecans, Osgood-Schlatter's disease, rheumatoid arthritis and the like. Among them, osteoarthritis and rheumatoid arthritis are preferably targeted.

Hereafter, an MMP activity lowering effect in the case of using a joint disease treating apparatus of the present invention will be explained with examples.

As an animal model of disease, a groove model [Lafeber et al., Orthop Res. Soc. Meeting 43, 462 (1998)] which had been developed Lafeber et al., was used. The groove model is an animal model of osteoarthritis, and it was produced as follows. At a loading part of the left hind-limb femoral condyle of a beagle (a female; a body weight of 10 to 20 kg), 0.5 mm crucial grooves are formed to form an operated knee. The right hind-limb was not operated. Subsequently, the right hind-limb was fixed with a bandage for 6 hr 3 times a week, an exercise load was applied on the operated knee to produce a groove-transformed joint disease model.

By using the animal models thus produced, low output ultrasonic waves are applied at the knee every day from the 10th week to the 20th week after the operation, and the effect of the ultrasonic waves was studied by analyzing tissue scores and measuring the MMP activities in synovia.

The following two groups of experiments were carried out.

### [Examples]

The ultrasonic group: a treating apparatus provided with an ultrasonic output unit having a built-in ultrasonic transducer which emits ultrasonic waves having ultrasonic output characteristics of a burst width of 200 µs, a frequency of 1.5 MHz, a repeating frequency of 1 kHz, and an intensity of 30 mW/cm² (SATA), was used. The ultrasonic output unit in a sate where ultrasonic waves were generated, was mounted, and the ultrasonic waves were applied on the knee of the left hind-limb of a beagle (n=5) of the above groove-transformed joint disease model for 20 minutes daily for 10 weeks. The left hind-limb was fixed in a state where it was bent at 90 degrees, the ultrasonic waves were applied in the direction toward the grooves of the loading part of the left hind-limb femoral condyle from the inner side of the knee. The ultrasonic transducer was brought into contact with the left hind-limb knee via an ultrasonic transmitting gel.

### [Comparative Examples]

The control group: an ultrasonic output unit in a state where ultrasonic waves were not generated was mounted on the hind-limb knee of a beagle (n=5) for 20 minutes a day everyday for 10 weeks.

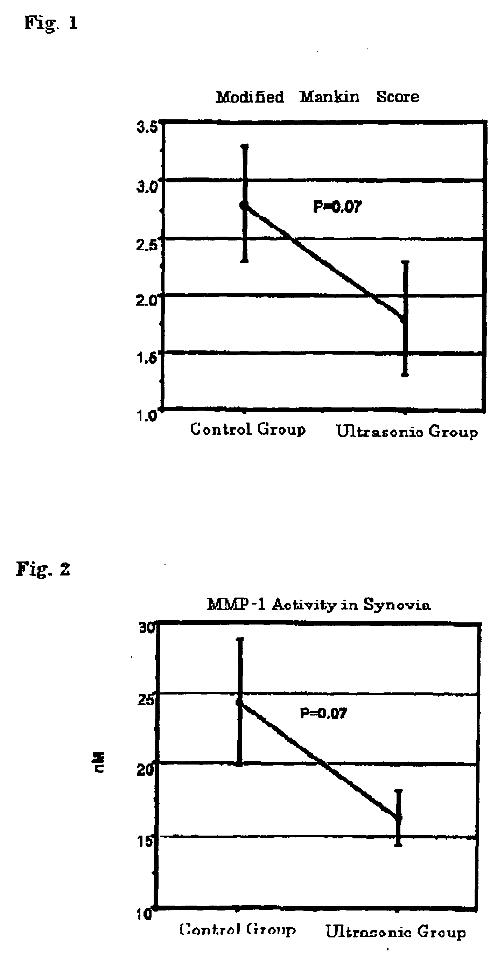

On the next day of the completion of a 10 week-ultrasonic application, the beagle was sacrificed, and slices of the femoral condyle tissue of the knee of the beagle were prepared. They were analyzed according to the modified method [Lafeber et al., Am. J. Pathol., 140, 1421-1429 (1992)] of Mankin score [Mankin et al., J. Bone and Joint Surg. 53-A, 523-537 (1971)]. Precisely, the tissue slices were dyed with safranin-O, and three items of a cartilaginous tissue structure, a distribution and a density of cartilaginous cells, and dyeability with safranin-O were evaluated under an optical microscope based on a 11-point scale shown in Table 1. The results are shown in Fig. 1.

**Table 1:**

| [A score table of Mankin evaluation method] | | | |
|---|---|---|---|
| Evaluation item | | Score | |
| I. Cartilaginous tissue structure | | | |
| a. | Normal | = | 0 |
| b. | Surface irregularities | = | 1 |
| c. | Clefts to transitional zone | = | 2 |
| d. | Clefts to radial zone | = | 3 |
| e. | Complete tissue disorganization | = | 4 |

| II. Distribution and density of cartilaginous cell | | | |
|---|---|---|---|
| a. | Normal | = | 0 |
| b. | Diffuse hypercellularity | = | 1 |
| c. | Cloning | = | 2 |
| d. | Hypocellularity | = | 3 |

| III. Dveabilitv with safranin-O | | | |
|---|---|---|---|
| a. | Normal | = | 0 |
| b. | Slight reduction | = | 1 |
| c. | Moderate reduction | = | 2 |
| d. | Severe reduction | = | 3 |
| e. | No dye noted | = | 4 |
| | Total score | | 11 |

Fig. 1 shows that low intensity ultrasonic pulses have a tendency to improve a tissue score in the femoral condyle (P=0.07).

Further, synovia samples were collected from the knee joints of the groove-modified joint disease model beagles of the ultrasonic application group and the control group, respectively, and activities of MMP-1, MMP-3 and MMP-13 in each of the synovia were determined by a MMP activity method [Beekman et al., FEBS Letters, 390, 221-225 (1996)] using a fluorogenic substrate. MMP activities in the synovia were determined by measuring MMP concentrations (nM) in the synovia. It is carried out by using TNO 113 (MMP-1 measuring substrate, manufactured by TNO Co.), TNO 003 (MMP-3 measuring substrate, manufactured by TNO Co.) or TNO013 (MMP-13 measuring substrate, manufactured by TNO Co.), as a substrate whose fluorescence intensities are increased due to its specific decomposition by each MMP, determining a fluorescence intensity and applying the fluorescence intensity on a standard curve prepared from a MMP of known concentrations.

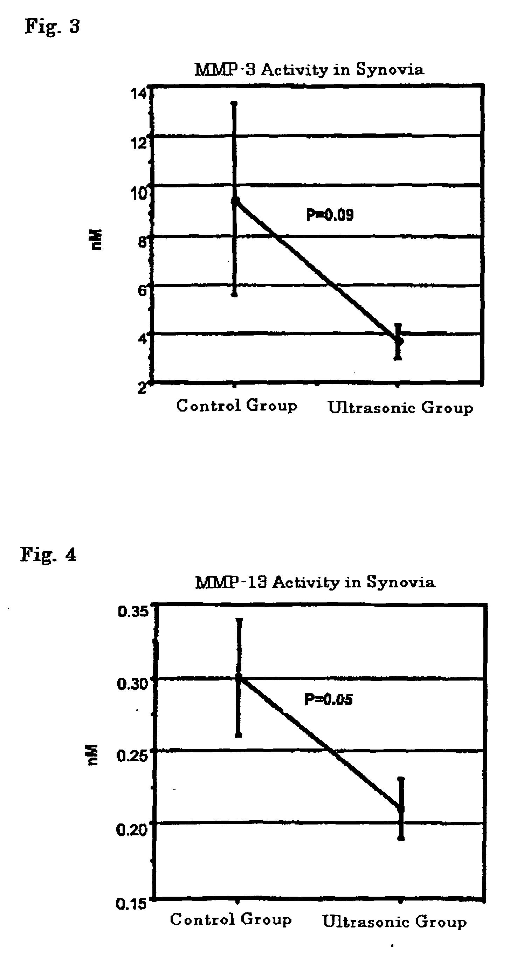

Figures 2 to 4 show that the activities of MMP-1, MMP-3 and MMP-13 of the ultrasonic wave application group had decreasing tendencies when compared with the activities of the control group (P=0.07, 0.09 and 0.05, respectively).

Further, in an in vitro experimental system, that is, in a coculture of cartilages derived from a normal human or an osteoarthritis (OA) patient, and monocytes (MNC) derived from synovia of an OA patient or a rheumatoid arthritis (RA) patient, an MMP-1 activity-decreasing effect was studied under same ultrasonic wave application conditions. This experiment also showed a significant decreasing effect on MMP-1 activity in a system using cartilaginous cells derived from an osteoarthritis patient.

According to the present invention, the enhanced MMP activity accompanied by a joint disease is decreased by using an ultrasonic radiating apparatus to improve a tissue score. Accordingly, a patient can treat a joint disease by applying ultrasonic waves for a short time of 20 minutes a day at home to repair degenerated tissue.

## Claims

1. A joint disease treating apparatus which is an apparatus for lowering an MMP activity at a joint site by applying ultrasonic waves, and which is a means provided with at least an ultrasonic transducer, an ultrasonic generator and a means for mounting the ultrasonic transducer on the joint site, and applying ultrasonic waves having a frequency of 1.3 to 2 MHz, a repeating frequency of 100 to 1,000 Hz, a burst width of 10 to 2,000 µ s and an intensity of 100 mW/cm² or less (SATA: Spatial Average-Temporal Average).

2. A method for treating joint diseases **characterized in that** ultrasonic waves are applied on a site where a matrix metalloprotease (MMP) activity is enhanced.

3. A method for treating joint diseases described in Claim 2 **characterized in that** the ultrasonic waves have characteristics of a frequency of 1.3 to 2 MHz, a repeating frequency of 100 to 1,000 Hz, a burst width of 10 to 2,000 µs and an intensity of 100 mW/cm² or less (SATA: Spatial Average-Temporal Average).

4. A method for treating joint diseases described in Claims 2 and 3 **characterized in that** the MMP is MMP-1, MMP-3 and/or MMP-13.
